# EUROPÄISCHE PATENTSCHRIFT

(11) **EP 1 091 804 B1**
(45) Veröffentlichungstag und Bekanntmachung des Hinweises auf die Patenterteilung: **27.02.2002**
(21) Anmeldenummer: 99927883.1
(22) Anmeldetag: 04.06.1999
(51) Int. Cl.: B01J 31/18, C07C 253/10, C07F 9/48

(54) **KATALYSATOR, UMFASSEND EINEN KOMPLEX EINES METALLS DER VIII. NEBENGRUPPE AUF BASIS EINES ZWEIZÄHNIGEN PHOSPHONITLIGANDEN UND VERFAHREN ZUR HERSTELLUNG VON NITRILEN**
CATALYST COMPRISING A COMPLEX OF A METAL FROM SUBGROUP VIII BASED ON A BIDENTATE PHOSPHONITE LIGAND, AND METHOD FOR PRODUCING NITRILES
CATALYSEUR COMPRENANT UN COMPLEXE D'UN METAL DU VIIIEME GROUPE SECONDAIRE A BASE D'UN LIGAND PHOSPHONITE A DEUX DENTS ET PROCEDE DE PREPARATION DE NITRILES

(30) Priorität: 05.06.1998 DE 19825212
(43) Veröffentlichungstag der Anmeldung: 18.04.2001
(73) Patentinhaber: BASF AKTIENGESELLSCHAFT, 67056 Ludwigshafen (DE)
(72) Erfinder: FISCHER, Jakob, D-85414 Kirchdorf (DE); SIEGEL, Wolfgang, D-67117 Limburgerhof (DE); KEITEL, Dagmar, Pascale, D-67117 Limburgerhof (DE); SIGGEL, Lorenz, D-69121 Heidelberg (DE)
(74) Vertreter: Kinzebach, Werner, Dr.
(86) Internationale Anmeldenummer: EP9903888
(87) Internationale Veröffentlichungsnummer: WO9964155

(56) Entgegenhaltungen:
- EP-A- 0 312 659
- WO-A-96/22968
- WO-A-96/33969
- WO-A-97/36856
- WO-A-98/43935
- WO-A-99/46044
- DE-A- 19 523 335
- DE-A- 19 740 180
- US-A- 5 360 938
- Baker, M.J.; Pringle, P.G. "Chiral Aryl Diphosphites: a New Class of Ligands for Hydrocyanation Catalysis" J. Chem. Soc., Chem. Commun. 1991, Seiten 1292-1293
- Huthmacher, K.; Krill, S. "Reactions with Hydrogen Cyanide (Hydrocyanation)" in: "Applied Homogenous Catalysis with Organometallic Compounds", Bd. 1, Cornils, B.; Herrmann, W.A. (Hrsg.), VCH Weinheim 1996, Seiten 465-486

## Beschreibung

Die vorliegende Erfindung betrifft einen Katalysator, der einen Komplex eines Metalls der VIII. Nebengruppe umfasst, welcher mindestens einen zweizähnigen Phosphonitliganden umfasst, ein Verfahren zur Herstellung von Gemischen monoolefinischer C₅-Mononitrile und ein Verfahren zur Herstellung von Adipodinitril durch katalytische Hydrocyanierung in Gegenwart eines solchen Katalysators.

Zur großtechnischen Herstellung von Polyamiden besteht weltweit ein großer Bedarf an α,ω-Alkylendiaminen, welche dabei als ein wichtiges Ausgangsprodukt dienen. α,ω-Alkylendiamine, wie z. B. das Hexamethylendiamin, werden fast ausschließlich durch Hydrierung der entsprechenden Dinitrile gewonnen. Fast alle großtechnischen Wege zur Herstellung von Hexamethylendiamin sind daher im Wesentlichen Varianten der Herstellung des Adipodinitrils, von dem jährlich weltweit etwa 1,0 Mio. Tonnen produziert werden.

In K. Weissermel, H.-J. Arpe, Industrielle Organische Chemie, 4. Auflage, VCH Weinheim, S. 266 ff. sind vier prinzipiell unterschiedliche Routen zur Herstellung von Adipinsäuredinitril beschrieben, u. a. die direkte Hydrocyanierung von 1,3-Butadien mit Cyanwasserstoff. Nach dem letztgenannten Verfahren erhält man in einer ersten Stufe durch Monoaddition ein Gemisch isomerer Pentennitrile, das in einer zweiten Stufe zu vorwiegend 3- und 4-Pentennitril isomerisiert wird. Anschließend wird in einer dritten Stufe durch anti-Markownikow-Cyanwasserstoffaddition an 4-Pentennitril das Adipinsäuredinitril gebildet.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin-, Phosphit- und Phosphinit-Komplexen des Nickels und Palladiums verwendet. Zur Herstellung von Adipinsäuredinitril durch Hydrocyanierung von Butadien werden vorwiegend Nickel(0)-Phosphitkatalysatoren, ggf. in Gegenwart einer Lewis-Säure als Promotor verwendet.

In J. Chem. Soc., Chem. Commun., 1991, S. 1292, werden chirale Aryldiphosphite als Liganden für Hydrocyanierungskatalysatoren beschrieben. Bei diesen Liganden ist die Phosphitgruppe über zwei ihrer Sauerstoffatome an die 3- und 3'-Positionen einer 2,2'-Binaphthyleinheit gebunden, mit der sie so einen 7-gliedrigen Heterocyclus bildet. Zusätzlich können zwei dieser Heterocyclen ebenfalls über eine 2,2'-Binaphthyleinheit zu einem zweizähnigen Chelatliganden verknüpft sein. In J. Chem. Soc., Chem. Commun., 1991, S. 803 f., werden dazu analoge Chelatdiphosphit-Komplexe von Nickel(0) und Platin(0) beschrieben, wobei als verbrückende Gruppe anstelle einer 2,2'-Binaphthyleinheit eine 2,2'-Biphenyleinheit eingesetzt wird.

Die US-A-5,449,807 beschreibt ein Verfahren zur Gasphasenhydrocyanierung von Diolefinen in Gegenwart eines geträgerten Nickelkatalysators auf Basis wenigstens eines zweizähnigen Phosphitliganden, wobei die beiden Phosphitgruppen durch eine unsubstituierte oder substituierte 2,2'-Biphenylgruppe verbrückt sind. Die US-A-5,440,067 beschreibt ein Verfahren zur Gasphasenisomerisierung von 2-Alkyl-3-monoalkennitrilen zu linearen 3- und/oder 4-Monoalkennitrilen in Gegenwart der in der US-A-5,449,807 beschriebenen Katalysatoren.

Die WO 95/14659 beschreibt ein Verfahren zur Hydrocyanierung von Monoolefinen, wobei Katalysatoren auf Basis von nullwertigem Nickel und zweizähnigen Phosphitliganden eingesetzt werden. Bei diesen Liganden sind die Phosphitgruppen zusammen mit zwei ihrer Sauerstoffatome Teil eines Aryl-aneilierten 7-gliedrigen Heterocyclus. Je zwei dieser Phosphitgruppen sind dann über die Sauerstoffatome, die nicht Teil des Heterocyclus sind, durch Aryl-anellierte Alkylengruppen verbrückt.

Die US-A-5,512,695 beschreibt ebenfalls ein Verfahren zur Hydrocyanierung von Monoolefinen in Gegenwart eines Nickelkatalysators, der einen zweizähnigen Phosphitliganden umfasst.

Die WO 96/11182 beschreibt ein Verfahren zur Hydrocyanierung in Gegenwart eines Nickelkatalysators auf Basis eines zwei- oder mehrzähnigen Phosphitliganden, bei dem die Phosphitgruppen nicht Bestandteil eines Heterocyclus sind. Die zur Verbrückung der Phosphitgruppen eingesetzten Gruppen entsprechen den in der WO 95/14659 beschriebenen.

Die US-A-5,523,453 beschreibt ein verfahren zur Hydrocyanierung in Gegenwart eines Nickelkatalysators auf Basis eines zweizähnigen Liganden, der mindestens eine Phosphinitgruppe und eine weitere phosphorhaltige Gruppe, die ausgewählt ist unter Phosphiniten und Phosphiten, umfasst. Die beiden phosphorhaltigen Gruppen dieser zweizähnigen Liganden sind wiederum über Aryl-anellierte Gruppen verbrückt. Die WO 97/23446 beschreibt ein Verfahren zur Hydrocyanierung von Diolefinen sowie zur Isomerisierung von 2-Alkyl-3-monoalkennitrilen in Gegenwart von Katalysatoren, die den in der US-A-5,523,453 beschriebenen entsprechen.

Die WO 96/22968 beschreibt ebenfalls ein Verfahren zur Hydrocyanierung diolefinischer Verbindungen und zur Isomerisierung der resultierenden, nichtkonjugierten 2-Alkyl-3-monoalkennitrile, wobei ein Nickel(0)-Katalysator auf Basis eines mehrzähnigen Phosphitliganden in Gegenwart einer Lewis-Säure als Promotor eingesetzt wird. Die Phosphitgruppen dieser mehrzähnigen Liganden sind dabei wiederum Bestandteile von Aryl-anellierten Heterocyclen und gegebenenfalls über Aryl-anellierte Gruppen verbrückt.

Keine der zuvor genannten Literaturstellen beschreibt Hydrocyanierungskatalysatoren auf Basis von Phosphonitliganden. Insbesondere werden keine Katalysatoren auf Basis von zweizähnigen Chelatphosphoniten beschrieben.

Die US-A 3,766,237 beschreibt ein Verfahren zur Hydrocyanierung ethylenisch ungesättigter Verbindungen, welche weitere funktionelle Gruppen, wie z. B. Nitrile, aufweisen können, in Gegenwart eines Nickelkatalysators. Diese Nickelkatalysatoren tragen vier Liganden der allgemeinen Formel M(X,Y,Z), wobei X, Y und Z unabhängig voneinander für einen Rest R oder OR stehen und R ausgewählt ist unter Alkyl- und Arylgruppen mit bis zu 18 Kohlenstoffatomen. Dabei werden jedoch nur Phosphine und Phosphite explizit genannt und in den Beispielen für die Hydrocyanierung eingesetzt. Dagegen ist nicht offenbart, daß Phosphonite als Liganden für Nickel(0)-Hydrocyanierungskatalysatoren eingesetzt werden können. Insbesondere werden keine zweizähnigen Chelatphosphonitliganden beschrieben.

Das Dokument DE-A-195 23 335 offenbart strukturell ähnliche zweizähnige Liganden mit dreiwertigem Phosphor, bevorzugt Phosphonit-Liganden, sowie deren Palladium Komplexe für Hydroformylierungen. In den Phosphonitliganden sind die beiden Phosphoratome ausschließlich über einen Kohlenwasserstoff-Spacer (C₆-C₃₀ (annelliert/substituiert) Alkylen, z.B. C₂, C₃, C₄, C₅, C₆) C,C verbrückt, d.h. keines der jeweils beiden Sauerstoffatome am Phosphor ist an der Verbrückung der beiden Phosphoratome beteiligt. Die nicht-phosphorverbrückenden Substituenten an den jeweiligen Sauerstoffatomen können dabei entweder zusammengenommen α,ω-bisannellierte (substituierte) zweiwertige Alkylenreste ausbilden, welches jeweils einen 7-10-gliedrigen P,O,O-Heterozyklus ergibt, oder jeweils (alkylsubstituierte) Arylreste darstellen. Das Dokument US-A-5 360 938 offenbart dreiwertige Phosphorliganden, u.a. in allgemeiner Form Phosphonite, für asymmetrische Synthesen, u.a. Hydrocyanierungen. Die beiden Phosphoratome können im Rahmen der allgemeinen Formel über einen (substituierten) Kohlenwasserstoff-Spacer und jeweils ein Sauerstoffatom O,O verbrückt sein, wobei die verbleibenden Substituenten am jeweiligen Phosphor nicht-verbrückt, d.h. P,O,C-Heterozyklusfrei sein können.

Der vorliegenden Erfindung liegt die Aufgabe zugrunde, neue Katalysatoren auf Basis eines Metalls der VIII. Nebengruppe zur Verfügung zu stellen. Diese sollen vorzugsweise bei der Hydrocyanierung von 1,3-Butadien und 1,3-Butadien-haltigen Kohlenwasserstoffgemischen eine gute Selektivität und eine gute katalytische Aktivität aufweisen. Bevorzugt sollen sie sich auch zur katalytischen Isomerisierung von Monoalkennitrilen und zur Zweitaddition von Cyanwasserstoff an diese, z. B. zur Herstellung von Adipodinitril, eignen.

Überraschenderweise wurden nun Katalysatoren auf Basis eines Metalls der VIII. Nebengruppe gefunden, welche mindestens einen zweizähnigen Phosphonitliganden umfassen.

Gegenstand der Erfindung ist daher ein Katalysator, umfassend einen Komplex eines Metalls der VIII. Nebengruppe, mit einem zweizähnigen Phosphonitliganden der allgemeinen Formel I 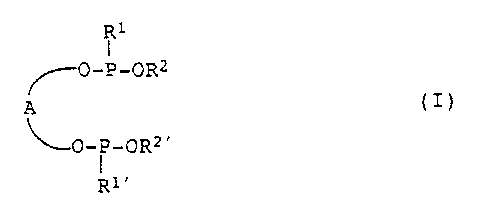 worin
- A: für eine C₂- bis C₇-Alkylenbrücke steht, die 1, 2 oder 3 Doppelbindungen und/oder 1, 2 oder 3 Substituenten, die ausgewählt sind unter Alkyl, Cycloalkyl und Aryl, aufweisen kann, wobei der Arylsubstituent zusätzlich 1, 2 oder 3 Substituenten, die ausgewählt sind unter Alkyl, Alkoxy, Halogen, Trifluormethyl, Nitro, Alkoxycarbonyl oder Cyano, tragen kann, und/oder die C₂- bis C₇-Alkylenbrücke durch 1, 2 oder 3 nicht benachbarte, gegebenenfalls substituierte Heteroatome unterbrochen sein kann, und/oder die C₂- bis C₇-Alkylenbrücke ein-, zwei- oder dreifach mit Aryl und/oder Hetaryl anelliert sein kann, wobei die anellierten Aryl- und Hetarylgruppen je 1, 2 oder 3 Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen,
- R¹ und R^{1'}: unabhängig voneinander für Alkyl, Cycloalkyl, Aryl oder Hetaryl stehen, welche je 1, 2 oder 3 Substituenten, die ausgewählt sind unter Alkyl, Cycloalkyl und Aryl, tragen können,
- R² und R^{2'}: unabhängig voneinander für Alkyl, Cycloalkyl, Aryl oder Hetaryl stehen, wobei die Aryl- und Hetarylgruppen je 1, 2 oder 3 Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E^{2,} tragen können, wobei E¹ und E² die zuvor angegebenen Bedeutungen besitzen können,
oder Salze und Mischungen davon.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₈-Alkyl-, bevorzugter C₁-C₆-Alkyl- und besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl.

Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituen-cen, ausgewählt unter Alkyl, Alkoxy, Halogen oder Trifluormethyl, auf.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl. Wenn die Arylgruppe substituiert ist, so weist sie bevorzugt 1, 2, 3, 4 oder 5, besonders bevorzugt 1, 2 oder 3 und speziell 1 oder 2 Substituenten in beliebigen Positionen auf.

Hetaryl steht vorzugsweise für Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, Halogen oder Trifluormethyl auf.

Die obigen Ausführungen zu Alkyl-, Cycloalkyl- und Arylresten gelten entsprechend für Alkoxy-, Cycloalkyloxy- und Aryloxyreste.

Die Reste NE¹E² stehen vorzugsweise für N,N-Dimethyl, N,N-Diethyl, N,N-Dipropyl, N,N-Diisopropyl, N,N-Di-n-butyl, N,N-Di-tert.-butyl, N,N-Dicyclohexyl oder N,N-Diphenyl.

Halogen steht für Fluor, Chlor, Brom und Iod, bevorzugt für Fluor und Chlor.

Bei den Phosphonitliganden der allgemeinen Formel I sind die Reste R¹ und R² bzw. R^{1'} und R^{2'} nicht miteinander verbrückt.

Der Rest A steht vorzugsweise für eine C₂- bis C₇-Alkylenbrücke, die 1-, 2- oder 3fach mit Aryl anelliert ist und die zusätzlich einen Substituenten, der ausgewählt ist unter Alkyl, Cycloalkyl und gegebenenfalls substituiertem Aryl, aufweisen kann und/oder die zusätzlich durch ein gegebenenfalls substituiertes Heteroatom unterbrochen sein kann.

Bei den anellierten Arylen der Reste A handelt es sich bevorzugt um Benzol oder Naphthalin. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, Trifluormethyl, Nitro, Carboxyl, Alkoxycarbonyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Anellierte Naphthaline, die im anellierten Ring substituiert sind, weisen vorzugsweise einen Substituenten in ortho-Position zur Phosphonitgruppe auf. Dieser steht dann bevorzugt für Alkyl oder Alkoxycarbonyl. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl. Halogen steht dabei insbesondere für Fluor und Chlor.

Wenn die C₂- bis C₇-Alkylenbrücke des Restes A durch 1, 2 oder 3, gegebenenfalls substituierte Heteroatome unterbrochen ist, so sind diese ausgewählt unter O, S oder NR⁵, wobei R⁵ für Alkyl, Cycloalkyl oder Aryl steht. Vorzugsweise ist die C₂- bis C₇-Alkylenbrücke des Restes A durch ein gegebenenfalls substituiertes Heteroatom unterbrochen.

Wenn die C₂- bis C₇-Alkylenbrücke des Restes A substituiert ist, so weist sie 1, 2 oder 3, insbesondere 1 Substituenten auf, der/die ausgewählt ist/sind unter Alkyl, Cycloalkyl und Aryl, wobei der Arylsubstituent zusätzlich 1, 2 oder 3 Substituenten, die ausgewählt sind unter Alkyl, Alkoxy, Halogen, Trifluormethyl, Nitro, Alkoxycarbonyl und Cyano, tragen kann. Vorzugsweise weist die Alkylenbrücke A einen Substituenten auf, der ausgewählt ist unter Methyl, Ethyl, Isopropyl, Phenyl, p-(C₁- bis C₄-Alkyl)phenyl, bevorzugt p-Methylphenyl, p-(C₁- bis C₄-Alkoxy)phenyl, bevorzugt p-Methoxyphenyl, p-Halogenphenyl, bevorzugt p-Chlorphenyl und p-Trifluormethylphenyl.

Vorzugsweise steht der Rest A für eine C₄- bis C₇-Alkylenbrücke, die wie zuvor beschrieben anelliert und/oder substituiert und/oder durch gegebenenfalls substituierte Heteroatome unterbrochen ist. Insbesondere steht der Rest A für eine C₄- bis C₅-Alkylenbrücke, die ein- oder zweifach mit Phenyl und/oder Naphthyl anelliert ist, wobei die Phenyl- oder Naphthylgruppen 1, 2 oder 3, insbesondere 1 oder 2 der zuvor genannten Substituenten tragen können.

Insbesondere steht A für einen Rest der Formeln II.1 bis II.5 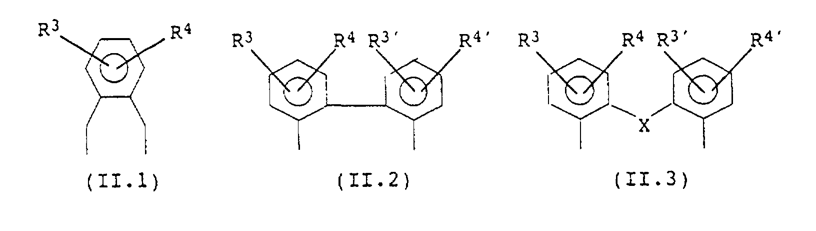 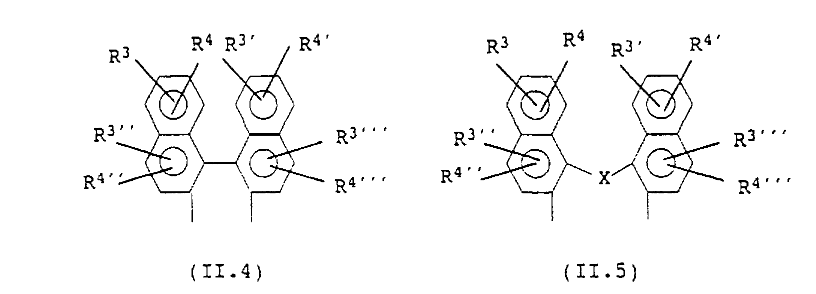 worin
- X: für O, S, NR⁵ steht, wobei
R⁵ für Alkyl, Cycloalkyl oder Aryl steht,
oder X für eine C₁- bis C₃-Alkylenbrücke steht, die eine Doppelbindung und/oder einen Alkyl-, Cycloalkyl- oder Arylsubstituenten aufweisen kann, wobei der Arylsubstituent 1, 2 oder 3 Substituenten, die ausgewählt sind unter Alkyl, Alkoxy, Halogen, Trifluormethyl, Nitro, Alkoxycarbonyl oder Cyano, tragen kann,
oder X für eine C₂- bis C₃-Alkylenbrücke steht, die durch O, S oder NR⁵ unterbrochen ist,
- R³, R^{3'}, R^{3''}, R^{3'''}, R⁴, R^{4'}, R^{4''} und R^{4'''}: unabhängig voneinander für Wasserstoff, Alkyl, Alkoxy, Halogen, Trifluormethyl, Nitro, Alkoxycarbonyl oder Cyano stehen.

Vorzugsweise steht A für einen Rest der Formel II.1, worin R³ und R⁴ für Wasserstoff stehen.

Vorzugsweise steht A für einen Rest der Formel II.2a 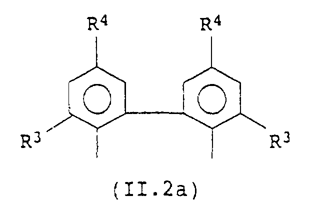 worin
- R³: für Wasserstoff oder C₁- bis C₄-Alkyl, bevorzugt Methyl, Isopropyl oder tert.-Butyl, steht,
- R⁴: für Wasserstoff, C₁- bis C₄-Alkyl, bevorzugt Methyl, Isopropyl oder tert.-Butyl, C₁- bis C₄-Alkoxy, bevorzugt Methoxy, Fluor, Chlor oder Trifluormethyl, steht.

Vorzugsweise steht A für einen Rest der Formel II.3a 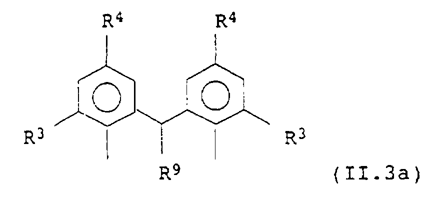 worin
- R³ und R⁴: die zuvor bei der Formel II.2a angegebenen Bedeutungen besitzen,
- R⁹: für Wasserstoff, C₁- bis C₄-Alkyl, bevorzugt Methyl oder Ethyl, Phenyl, p-(C₁- bis C₄-Alkoxy)phenyl, bevorzugt p-Methoxyphenyl, p-Fluorphenyl, p-Chlorphenyl oder p-(Trifluormethyl)phenyl steht.

Vorzugsweise steht A für einen Rest der Formel II.4, worin R³, R^{3'}, R^{3''}, R^{3'''}, R⁴, R^{4'}, R^{4''} und R^{4'''} für Wasserstoff stehen.

Vorzugsweise steht A für einen Rest der Formel II.4, worin R³, R^{3'}, R⁴, R^{4'}, R^{4''} und R^{4'''} für Wasserstoff stehen und die Reste R^{3''} und R^{3'''} unabhängig voneinander für Alkoxycarbonyl, bevorzugt Methoxy-, Ethoxy-, n-Propyloxy- oder Isopropyloxycarbonyl, stehen. Insbesondere stehen die Reste R^{3''} und R^{3'''} in ortho-Position zur Phosphonitgruppe.

Vorzugsweise steht A für einen Rest der Formel II.5, worin R³, R^{3'}, R^{3''}, R^{3'''}, R⁴, R^{4'}, R^{4''} und R^{4'''} für Wasserstoff stehen und X für CR⁹ steht, wobei R⁹ die zuvor angegebenen Bedeutungen besitzt.

Vorzugsweise steht A für einen Rest der Formel II.5, worin R³, R^{3'}, R⁴, R^{4'}, R^{4''} und R^{4'''} für Wasserstoff stehen, X für CR⁹ steht und die Reste R^{3''} und R^{3'''} unabhängig voneinander für Alkoxycarbonyl, bevorzugt Methoxy-, Ethoxy-, n-Propyloxy- oder Isopropyloxycarbonyl, stehen. Insbesondere stehen die Reste R^{3''} und R^{3'''} in ortho-Position zur Phosphonitgruppe.

Vorzugsweise stehen in der Formel I die Reste R¹ und R^{1'} unabhängig voneinander für Alkyl oder Aryl, insbesondere Phenyl, 1-Naphthyl oder 2-Naphthyl.

Vorzugsweise stehen R² und R^{2'} unabhängig voneinander für Phenylsubstituenten, die gegebenenfalls 1 oder 2 Substituenten, die ausgewählt sind unter Alkyl, Alkoxy, Halogen, Trifluormethyl, Nitro, Cyano, Alkoxycarbonyl oder Carboxyl, tragen können.

Nach einer bevorzugten Ausführungsform ist der Phosphonitligand der Formel I ausgewählt unter Liganden der Formeln Ia bis Ic 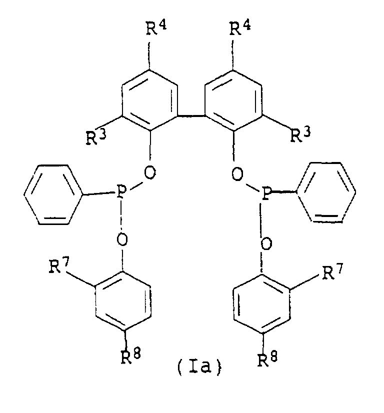 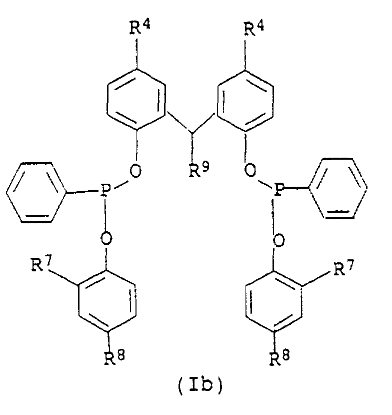 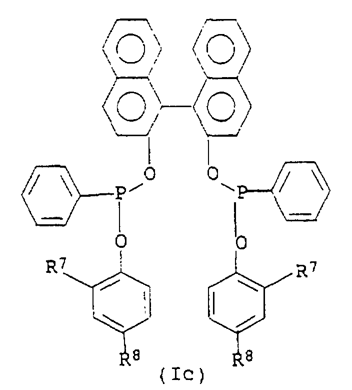 wobei in der Formel Ia die Substituenten R³, R⁴, R⁷ und R⁸ die folgenden Bedeutungen besitzen:

| R³ | R⁴ | R⁷ | R⁸ |
|---|---|---|---|
| H | H | H | H |
| tert.-Butyl | Methyl | H | H |
| tert.-Butyl | Methoxy | H | H |
| H | H | Methyl | H |
| H | H | Ethyl | H |
| H | H | Isopropyl | H |
| H | H | tert.-Butyl | H |
| H | Cl | H | H |
| H | CF₃ | H | H |
| H | H | Methyl | Methoxy |

in der Formel Ib die Substituenten R⁴, R⁷, R⁸ und R⁹ die folgenden Bedeutungen besitzen:

| R⁴ | R⁷ | R⁸ | R⁹ |
|---|---|---|---|
| H | H | H | H |
| Cl | H | H | H |
| Methoxy | H | H | H |
| H | H | H | Phenyl |
| H | Methyl | H | H |
| H | Methyl | Methoxy | H |
| H | Methyl | Methoxy | Phenyl |

in der Formel Ic die Substituenten R⁷ und R⁸ die folgenden Bedeutungen besitzen:

| R⁷ | R⁸ |
|---|---|
| H | H |
| Methyl | H |
| Ethyl | H |
| Isopropyl | H |
| tert.Butyl | H |
| Methyl | Methoxy |
| i-Propyl | H |
| i-Propyl | Methoxy |
| H | Cl |
| H | CF₃ |

Ein weiterer Gegenstand der Erfindung sind Phosphonitliganden der allgemeinen Formel I 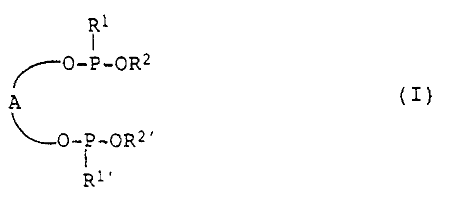 wie zuvor definiert, worin
- R² und R^{2'}: unabhängig voneinander für Alkyl, Cycloalkyl, Aryl oder Hetaryl stehen, wobei die Aryl- und Hetarylgruppen je 1 oder 2 Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E^{2,} tragen können, wobei E¹ und E² gleich oder verschieden sein können und für Alkyl, Cycloalkyl oder Aryl stehen.

Bevorzugt stehen R² und R^{2'} unabhängig voneinander für Phenylgruppen, die 1 oder 2 der zuvor genannten Substituenten tragen können.

Die erfindungsgemäßen Katalysatoren können einen oder mehrere der Phosphonitliganden der allgemeinen Formel I aufweisen. Zusätzlich zu den zuvor beschriebenen Liganden der allgemeinen Formel I können sie noch wenigstens einen weiteren Liganden, der ausgewählt ist unter Cyanid, Halogeniden, Aminen, Carboxylaten, Acetylaceton, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit-, Phosphonit- und Phosphitliganden aufweisen. Diese weiteren Liganden können ebenfalls ein-, zwei- oder mehrzähnig sein und an das Metall der VIII. Nebengruppe koordinieren. Geeignete weitere phosphorhaltige Liganden sind z. B. die zuvor als Stand der Technik beschriebenen Phosphin-, Phosphinit- und Phosphitliganden.

Bevorzugt handelt es sich bei dem Metall der VIII. Nebengruppe um Cobalt, Rhodium, Ruthenium, Palladium oder Nickel. Werden die erfindungsgemäßen Katalysatoren zur Hydrocyanierung eingesetzt, so handelt es sich bei dem Metall der VIII. Nebengruppe insbesondere um Nickel.

Zur Herstellung der in den erfindungsgemäßen Katalysatoren eingesetzten Phosphonitliganden der allgemeinen Formel I kann man gemäß dem folgenden Schema zunächst eine Dihalogenphosphor(III)verbindung III, worin R¹ (bzw. R^{1'}) die zuvor angegebenen Bedeutungen besitzt, mit einem Monoalkohol IV, worin R² (bzw. R^{2'}) die zuvor angegebenen Bedeutungen besitzt, zu einer Verbindung der Formel V umsetzen. Gewünschtenfalls kann diese Verbindung V vor der weiteren Umsetzung nach bekannten Verfahren isoliert und/oder gereinigt werden, z. B. durch Destillation. Die Verbindung V wird dann mit einem Diol der Formel VI zu den zweizähnigen Phosphonitliganden der Formel (I) umgesetzt. Für den Fall, dass in der Formel (I) R¹ = R^{1'} und R² = R^{2'} ist, können zwei Äquivalente der Formel V in einer einstufigen Reaktion mit einem Äquivalent der Formel VI umgesetzt werden. Ansonsten wird zunächst ein Äquivalent der Formel V mit einem Äquivalent der Formel VI umgesetzt und nach Bildung des Monokondensationsproduktes wird eine zweite Verbindung der Formel (V) Cl-PR^{1'}-OR^{2'} zugegeben und weiter zu dem Phosphonit der Formel (I) umgesetzt.

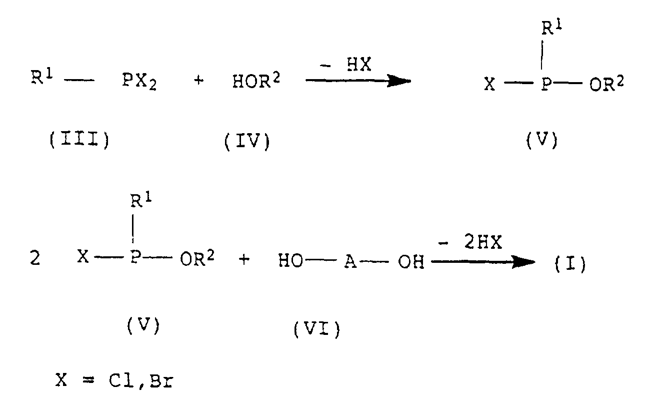

Bei der Verbindung der Formel (III) handelt es sich bevorzugt um eine Dichlorphosphor(III)verbindung. Geeignete Verbindungen mit den zuvor genannten Resten R¹ sind bekannt. Wenn z. B. R¹ für Phenyl steht, so handelt es sich um Dichlorphenylphosphin.

Geeignete Alkohole der Formel IV, worin R² die zuvor angegebenen Bedeutungen besitzt, sind ebenfalls bekannt. Geeignete aromatische Alkohole der Formel HOR² sind z. B. 2-tert-Butyl-4-methylphenol, 2-Isopropylphenol, 2-tert-Butylphenol, 4-tert-Butylphenol, 2,6-Di-tert-butyl-4-methylphenol, 2,4-Di-tert-butylphenol, 2,6-Di-tert-butylphenol, 2,4-Dimethylphenol, 2,5-Dimethylphenol, 2,6-Dimethylphenol, 3,4-Dimethylphenol, 3,5-Dimethylphenol, 2-Ethylphenol, 3-Ethylphenol, 4-Ethylphenol, 5-Isopropyl-2-methylphenol, m-Kresol, o-Kresol, p-Kresol, 1-Naphthol, 2-Naphthol, Phenol, 1-Brom-2-naphthol, 3-Bromphenol, 5-Chlorchinolin-8-ol, 4-Chlor-3,5-dimethylphenol, 2-Chlor-5-methylphenol, 4-Chlor-3-methylphenol, 2-Chlor-6-nitrophenol, 2-Clorphenol, 3-Chlorphenol, 4-Chlorphenol, 4-Chlorresorcin, 2,3-Dichlorphenol, 2,4-Dichlorphenol, 2,5-Dichlorphenol, 2,6-Dichlorphenol, 3,4-Dichlorphenol, 2-Fluorphenol, 3-Fluorphenol, 4-Fluorphenol, 3-Methyl-4-nitrophenol, 3-Isopropyl-4-nitrophenol, 3-Isopropyl-4-nitrophenol, 2-Nitroanisol, 4-Nitrobrenzcatechin, 2-Nitrophenol, 3-Nitrophenol, 2-Methoxy-3-methylphenol, 2-Methoxy-4-methylphenol, 2-Methoxyphenol, 3-Methoxyphenol, 4-Methoxyphenol. Bevorzugte Alkohole der Formel HOR¹ sind 2-Isopropylphenol, 2,6-Di-tert-butyl-4-methylphenol, 2,4-Di-tert-butylphenol, 2,6-Di-tert-butylphenol, Phenol, 2-Fluorphenol, 3-Fluorphenol, 4-Fluorphenol, 4-Nitrobrenzcatechin, 2-Methoxy-4-methylphenol, 2-Trifluormethylphenol, 3,5-Bis(trifluormethyl)phenol, 4-Cyanophenol, etc.

Geeignete Alkohole der Formel HO-A-OH, worin A die zuvor angegebenen Bedeutungen besitzt, sind bekannt. Dazu zählen z. B. Biphenyl-2,2'-Diol und Binaphthyl-2,2'-Diol. Weitere geeignete Diole werden in der US-A-5,312,996, Sp. 19 genannt, auf die hier Bezug genommen wird.

Sowohl die Umsetzung der Verbindung (III) mit (IV) zu (V) als auch die weitere Umsetzung zu den zweizähnigen Phosphonitliganden der allgemeinen Formel (I) verläuft im Allgemeinen bei einer erhöhten Temperatur im Bereich von etwa 40 bis etwa 200 °C. Beide Umsetzungen können in Gegenwart einer Base, z. B. einem aliphatischen Amin, wie Diethylamin, Propylamin, Dibutylamin, Trimethylamin, Tripropylamin und vorzugsweise Triethylamin oder Pyridin erfolgen. Bevorzugt erfolgt die Halogenwasserstoffabspaltung im ersten Reaktionsschritt rein thermisch.

Vorteilhafterweise gelingt die Herstellung der erfindungsgemäß eingesetzten Phosphonitliganden der Formel I ohne Verwendung von Magnesium- oder Lithium-organischen Verbindungen. Die einfache Reaktionssequenz erlaubt eine breite Variationsmöglichkeit der Liganden. Die Darstellung gelingt somit effizient und ökonomisch aus leicht zugängigen Edukten.

Zur Herstellung der erfindungsgemäßen Katalysatoren kann man mindestens einen Phosphonitliganden der Formel I mit einem Metall der VIII. Nebengruppe, z. B. Nickel, oder mit einer Verbindung des Metalls in Gegenwart eines Reduktionsmittels oder einem Komplex des Metalls jeweils in einem inerten Lösungsmittel zur Reaktion bringen. Geeignete Nickelverbindungen sind dabei z. B. Verbindungen, in denen das Übergangsmetall eine Oxidationsstufe höher als 0 einnimmt, und die bei der Umsetzung mit dem Phosphonitliganden der Formel I, gegebenenfalls in Gegenwart eines geeigneten Reduktionsmittels, in situ reduziert werden. Dazu zählen z. B. die Halogenide, bevorzugt die Chloride, und die Acetate der zuvor genannten Übergangsmetalle. Dabei wird bevorzugt NiCl₂ eingesetzt. Geeignete Reduktionsmittel sind z. B. Metalle, bevorzugt Alkalimetalle, wie Na und K, Aluminium, Zink sowie Trialkylaluminiumverbindungen.

Werden zur Herstellung der Phosphonit-Nickel(0)-Komplexe bereits Komplexverbindungen des Übergangsmetalls eingesetzt, so liegt in diesen das Übergangsmetall vorzugsweise bereits nullwertig vor. Bevorzugt werden zur Herstellung Komplexe mit Liganden eingesetzt, die den zuvor genannten, zusätzlichen Liganden der erfindungsgemäßen Komplexe entsprechen. In diesem Falle erfolgt die Herstellung durch teilweisen oder vollständigen Ligandenaustausch mit den zuvor beschriebenen Phosphonitliganden der Formel I.

Bevorzugt ist der Nickelkomplex Bis(1,5-cyclooctadien)nickel(0).

Geeignete inerte Lösungsmittel zur Herstellung der Nickel(0)-Komplexe sind beispielsweise Aromaten, wie Benzol, Toluol, Ethylbenzol, Chlorbenzol, Ether, vorzugsweise Diethylether und Tetrahydrofuran, oder Halogenalkane, beispielsweise Dichlormethan, Chloroform, Dichlorethan und Trichlorethan. Geeignete Lösungsmittel sind auch die flüssigen Edukte und/oder Produkte der katalysierten Reaktion. Die Temperatur liegt dabei in einem Bereich von -70 °C bis 150 °C, vorzugsweise von 0 °C bis 100 °C, besonders bevorzugt etwa bei Raumtemperatur.

Wird zur Herstellung der Phosphonit-Nickel(0)-Komplexe elementares Nickel eingesetzt, so liegt dieses vorzugsweise als Pulver vor. Die Umsetzung von Nickel und Phosphonitligand erfolgt vorzugsweise in einem Produkt der katalysierten Reaktion, wie der Hydrocyanierungsreaktion, als Lösungsmittel, z. B. in einem Gemisch monoolefinischer C₅-Mononitrile oder bevorzugt in 3-Pentennitril oder 2-Methyl-3-butennitril. Gegebenenfalls kann auch der Ligand als Lösungsmittel eingesetzt werden. Die Temperatur liegt in einem Bereich von etwa 0 bis 150 °C, bevorzugt 60 bis 100 °C.

Vorzugsweise liegt das Molmengenverhältnis von Metall der VIII. Nebengruppe zu zweizähnigem Phosphonitliganden in einem Bereich von etwa 1:1 bis 1:5, bevorzugt 1:1 bis 1:3.

Ein weiterer Gegenstand der Erfindung ist ein Verfahren zur Herstellung von Gemischen monoolefinischer C₅-Mononitrile mit nichtkonjugierter C=C- und C ≡ N-Bindung durch katalytische Hydrocyanierung von Butadien oder eines 1,3-Butadien-haltigen Kohlenwasserstoffgemisches, das dadurch gekennzeichnet ist, dass die Hydrocyanierung in Gegenwart mindestens eines der zuvor beschriebenen erfindungsgemäßen Katalysatoren erfolgt.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zu Adipodinitril geeignet sind, kann man reines Butadien oder 1,3-Butadien-haltige Kohlenwasserstoffgemische einsetzen.

Wird nach dem erfindungsgemäßen Verfahren ein Kohlenwasserstoffgemisch eingesetzt, so weist dieses einen 1,3-Butadien-Gehalt von mindestens 10 Vol.-%, bevorzugt mindestens 25 Vol.-%, insbesondere mindestens 40 Vol.-%, auf.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im Allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls vor der Hydrocyanierung von Alkinen, wie z. B. Propin oder Butin, von 1,2-Dienen, wie z. B. Propadien, und von Alkeninen, wie z. B. Vinylacetylen, im Wesentlichen befreit. Ansonsten werden u. U. Produkte erhalten, bei denen eine C=C-Doppelbindung in Konjugation mit der C≡N-Bindung steht. Diese können u. U. als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben können, insbesondere Alkine, 1,2-Diene und Gemische davon. Zur Entfernung dieser Komponenten wird der C₄-Schnitt vor der Addition von Cyanwasserstoff einer katalytischen Teilhydrierung unterzogen. Diese Teilhydrierung erfolgt in Gegenwart eines Hydrierungskatalysators, der befähigt ist, Alkine und 1,2-Diene selektiv neben anderen Dienen und Monoolefinen zu hydrieren.

Geeignete heterogene Katalysatorsysteme für die selektive Hydrierung sind bekannt und umfassen im Allgemeinen eine Übergangsmetallverbindung auf einem inerten Träger. Insbesondere handelt es sich dabei um die in den US-A-4,587,369; US-A-4,704,492 und US-A-4,493,906 beschriebenen, auf die hier in vollem Umfang Bezug genommen wird. Weitere geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierlich, semikontinuierlich oder diskontinuierlich erfolgen.

Geeignete Reaktoren für die Umsetzung sind dem Fachmann bekannt und werden z. B. in Ullmanns Enzyklopädie der technischen Chemie, Bd. 1, 3. Aufl., 1951, S. 743 ff. und S. 769 ff. beschrieben. Vorzugsweise wird für ein kontinuierliches Verfahren eine Rührkesselkaskade oder ein Rohrreaktor verwendet.

Erfolgt die Addition des Cyanwasserstoffs an 1,3-Butadien oder ein 1,3-Butadien-haltiges Kohlenwasserstoffgemisch semikontinuierlich oder diskontinuierlich, so wird für das erfindungsgemäße Verfahren z. B. ein Autoklav verwendet, der gewünschtenfalls mit einer Rührvorrichtung und einer Innenauskleidung versehen sein kann.

Ein geeignetes semikontinuierliches Verfahren umfasst:
a) Befüllen eines Reaktors mit 1,3-Butadien oder dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.
b) Umsetzung des Gemisches bei erhöhter Temperatur und erhöhtem Druck. Dabei liegt die Reaktionstemperatur im Allgemeinen in einem Bereich von etwa 0 bis 200°C, bevorzugt etwa 50 bis 150 °C. Der Druck liegt im Allgemeinen in einem Bereich von etwa 1 bis 200 bar, bevorzugt etwa 1 bis 100 bar, insbesondere 1 bis 50 bar, insbesondere bevorzugt 1 bis 20 bar. Dabei wird während der Reaktion Cyanwasserstoff nach Maßgabe seines Verbrauchs eingespeist.
c) Gegebenenfalls Vervollständigung des Umsatzes durch Nachreagieren und anschließende Aufarbeitung. Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfasst die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im Wesentlichen die bei dem semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Bevorzugt erfolgt die Addition des Cyanwasserstoffs an 1,3-Butadien oder ein 1,3-Butadien-haltiges Kohlenwasserstoffgemisch kontinuierlich. Die Reaktionsführung erfolgt dabei im Allgemeinen so, dass im Wesentlichen keine größeren nicht umgesetzten Cyanwasserstoffmengen im Reaktor vorhanden sind. Geeignete Verfahren zur kontinuierlichen Hydrocyanierung sind dem Fachmann bekannt. Dazu zählt z. B. ein Zulaufverfahren, bei dem 1,3-Butadien und Blausäure über getrennte Zuläufe nach Maßgabe ihres Verbrauchs einem Reaktor zugeführt werden. Der Katalysator kann dabei gemeinsam mit einem der Edukte oder über einen separaten Zulauf zugeführt werden. Geeignete, vorzugsweise gut durchmischbare Reaktoren sind dem Fachmann ebenfalls bekannt. Dazu zählen z. B. Rührkessel, Kesselkaskaden und Rohrreaktoren, die gegebenenfalls mit einer Innenauskleidung versehen sind. Vorzugsweise erfolgt auch die Aufarbeitung der Reaktionsprodukte nach einem üblichen kontinuierlichen Verfahren.

Im Allgemeinen beträgt das bei der Monoaddition von Cyanwasserstoff an 1,3-Butadien oder das 1,3-Butadien-haltige Kohlenwasserstoffgemisch unmittelbar nach Beendigung der Addition (kein nicht umgesetzter Cyanwasserstoff mehr vorhanden) erhaltene Mengenverhältnis von 3-Pentennitril zu 2-Methyl-3-butennitril mindestens 0,4:1. Vorteilhafterweise findet bei höheren Reaktionstemperaturen und/oder bei längeren Reaktionszeiten in Gegenwart der erfindungsgemäßen Katalysatoren zusätzlich eine Isomerisierung statt, wobei das erhaltene Mengenverhältnis von 3-Pentennitril zu 2-Methyl-3-butennitril dann im Allgemeinen etwa 2:1, bevorzugt 5:1, insbesondere 8:1, beträgt.

Allgemein lässt sich die Herstellung von Adipinsäuredinitril aus Butadien oder einem Butadien-haltigen Kohlenwasserstoffgemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril bzw. 4-Pentennitril und ein möglichst geringer Anteil an konjugiertem und gegebenenfalls als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird.

Vorteilhafterweise eignen sich die erfindungsgemäßen Katalysatoren auf Basis von Phosphonitliganden auch für die Stellungs- und Doppelbindungsisomerisierung in Schritt 2 und/oder die Zweitaddition von Cyanwasserstoff in Schritt 3.

Ein weiterer Gegenstand der Erfindung ist daher ein Verfahren zur katalytischen Isomerisierung verzweigter aliphatischer Monoalkennitrile mit nichtkonjugierter C=C- und C≡N-Bindung zu linearen Monoalkennitrilen, das dadurch gekennzeichnet ist, dass die Isomerisierung in Gegenwart eines erfindungsgemäßen Katalysators erfolgt.

Geeignete verzweigte aliphatische Monoalkennitrile sind vorzugsweise acyclische, aliphatische, nichtkonjugierte 2-Alkyl-3-Monoalkennitrile und insbesondere 2-Methyl-3-butennitril. Vorzugsweise werden zur Isomerisierung Gemische monoolefinischer C₅-Mononitrile eingesetzt, wie sie durch das zuvor beschriebene Verfahren zur katalytischen Hydrocyanierung von Butadien oder von 1,3-Butadien-haltigen Kohlenwasserstoffgemischen erhältlich sind. Vorteilhafterweise zeigen die erfindungsgemäßen Katalysatoren dabei eine gute Aktivität in Bezug auf die Bildung linearer Monoalkennitrile. Die Isomerisierung kann gewünschtenfalls in Gegenwart eines üblichen Promotors, z. B. einer Lewis-Säure, wie AlCl₃ oder ZnCl₂, erfolgen. Vorteilhafterweise ermöglichen die erfindungsgemäßen Katalysatoren im Allgemeinen eine Isomerisierung ohne Promotorzusatz. Dabei ist die Selektivität der erfindungsgemäßen Katalysatoren bei der Isomerisierung ohne Promotorzusatz im Allgemeinen höher als mit Promotorzusatz. Des weiteren kann auf eine aufwendige Abtrennung des Promotors nach der Isomerisierung verzichtet werden. Somit ist prinzipiell nur ein Katalysatorkreislauf für Hydrocyanierung, Isomerisierung und gegebenenfalls eine Zweitaddition von Cyanwasserstoff erforderlich. Die Einsparung des Promotors und die prinzipiell mögliche Vereinfachung der Prozessführung ermöglichen im Allgemeinen eine Senkung der Kosten gegenüber bekannten Verfahren.

Die Temperatur bei der Isomerisierung liegt in einem Bereich von etwa 50 bis 160 °C, bevorzugt 70 bis 130 °C.

Ein weiterer Gegenstand der Erfindung ist ein Verfahren zur Herstellung von Adipinsäuredinitril durch katalytische Hydrocyanierung von linearen monoolefinischen C₅-Mononitrilen, das dadurch gekennzeichnet ist, dass die Hydrocyanierung in Gegenwart eines erfindungsgemäßen Katalysators erfolgt. Vorteilhafterweise setzt man zur Hydrocyanierung ein Gemisch monoolefinischer C₅-Mononitrile ein, welches nach dem erfindungsgemäßen Verfahren zur katalytischen Hydrocyanierung von Butadien oder eines 1,3-Butadien-haltigen Kohlenwasserstoffgemisches erhältlich ist und welches gegebenenfalls zusätzlich einer Aufarbeitung und/oder einer Isomerisierung nach den zuvor beschrieben erfindungsgemäßen Isomerisierungsverfahren unterzogen wurde. Nach einer geeigneten Ausführungsform des erfindungsgemäßen Verfahrens erfolgt die Hydrocyanierung der monoolefinischen C₅-Mononitrile in Gegewanrt eines Promotors, z. B. einer Lewis-Säure, wie AlCl₃, ZnCl₂, BF₃, B(C₆H₅)₃, SnCl₄, Sn(C₆H₅)₃OSO₂CF₃ etc.

Nach einer geeigneten Ausführungsform des erfindungsgemäßen Verfahrens zur Herstellung von Adipinsäuredinitril erfolgt die katalytische Hydrocyanierung von Butadien oder eines 1,3-Butadien-haltigen Kohlenwasserstoffgemisches (Schritt 1) und die Isomerisierung (Schritt 2) im Sinne einer Eintopfreaktion ohne Isolierung der Hydrocyanierungsprodukte. Dabei können Hydrocyanierung und Isomerisierung z. B. in einem Reaktor erfolgen, wobei gegebenenfalls nach Beendigung der Cyanwasserstoffzugabe die Reaktionstemperatur erhöht wird. Hydrocyanierung und Isomerisierung können auch in separaten Reaktoren erfolgen, wobei z. B. nach Beendigung der Monoaddition von Cyanwasserstoff in einem ersten Reaktor das katalysatorhaltige Reaktionsgemisch ohne Isolierung und Aufarbeitung in einen zweiten Reaktor überführt und in diesem isomerisiert wird.

Nach einer weiteren geeigneten Ausführungsform des erfindungsgemäßen Verfahrens erfolgen alle drei Schritte der Adipinsäuredinitrilherstellung, d. h. Herstellung monoolefinischer C₅-Mononitrile, Isomerisierung und Zweitaddition von Cyanwasserstoff im Sinne einer Eintopfreaktion.

Gegenstand der Erfindung ist daher ein Verfahren zur Herstellung von Adipinsäuredinitril, umfassend
a) Herstellung eines Gemisches monoolefinischer C₅-Mononitrile mit nichtkonjugierter C=C- und C≡N-Bindung durch katalytische Hydrocyanierung von Butadien oder eines 1,3-Butadien-haltigen Kohlenwasserstoffgemisches,
b) katalytische Isomerisierung des Gemisches aus a),
c) katalytische Hydrocyanierung des isomerisierten Gemisches aus b),
das dadurch gekennzeichnet ist, dass die Schritte a), b) und c) in Gegenwart wenigstens eines erfindungsgemäßen Katalysators und ohne Isolierung des/der Produkte(s) aus Schritt a) und/oder b) erfolgen.

Die erfindungsgemäßen Katalysatoren lassen sich aus leicht zugängigen und zum Teil im Handel erhältlichen Vorstufen einfach und somit kostengünstig herstellen. Vorteilhafterweise zeigen sie eine hohe Aktivität und eine gute Selektivität im Bezug auf die bei der Hydrocyanierung von 1,3-Butadien-haltigen Kohlenwasserstoffgemischen erhaltenen Monoadditionsprodukte bzw. Isomerisierungsprodukte. Im Allgemeinen weisen sie eine höhere Stabilität gegenüber Cyanwasserstoff als übliche Hydrocyanierungskatalysatoren auf und können bei der Hydrocyanierung auch mit einem Überschuss an Cyanwasserstoff versetzt werden, ohne dass es zu einer merklichen Abscheidung von inaktiven Nickel(II)-Verbindungen, wie z. B. Nickel(II)-Cyanid, kommt. Im Gegensatz zu bekannten Hydrocyanierungskatalysatoren auf Basis nicht-komplexer Phosphin- und Phosphitliganden eignen sich die erfindungsgemäßen Katalysatoren somit nicht nur für kontinuierliche Hydrocyanierungsverfahren, bei denen ein Cyanwasserstoffüberschuss im Reaktionsgemisch im Allgemeinen wirkungsvoll vermieden werden kann, sondern auch für semikontinuierliche Verfahren und Batch-Verfahren, bei denen im Allgemeinen ein starker Cyanwasserstoffüberschuss vorliegt. Somit weisen die erfindungsgemäß eingesetzten Katalysatoren und die auf ihnen basierenden Verfahren zur Hydrocyanierung im Allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid srark giftig ist und unter hohen Kosten aufgearbeitet oder entsorgt werden muss. Zudem ist bei der Herstellung der erfindungsgemäßen Katalysatoren im Allgemeinen kein oder ein geringerer Überschuss von Ligand gegenüber Metall der VIII. Nebengruppe erforderlich als bei herkömmlichen Katalysatoren.

Neben der Hydrocyanierung von 1,3-Butadien-haltigen Kohlenwasserstoffgemischen eignen sich die Katalysatoren der Formel I im Allgemeinen für alle gängigen Hydrocyanierungsverfahren. Dabei sei insbesondere die Hydrocyanierung von nichtaktivierten Olefinen, z. B. von Styrol und 3-Pentennitril, genannt.

Die zuvor beschriebenen Katalysatoren, die chirale Phosphonitliganden der Formel I umfassen, eignen sich zur enantioselektiven Hydrocyanierung.

Die Erfindung wird anhand der folgenden, nicht einschränkenden Beispiele näher erläutert.

### Beispiele

In den Beispielen 1 und 3 wurde der folgende Ligand I, in den Beispielen 2 und 4 der Ligand II eingesetzt:

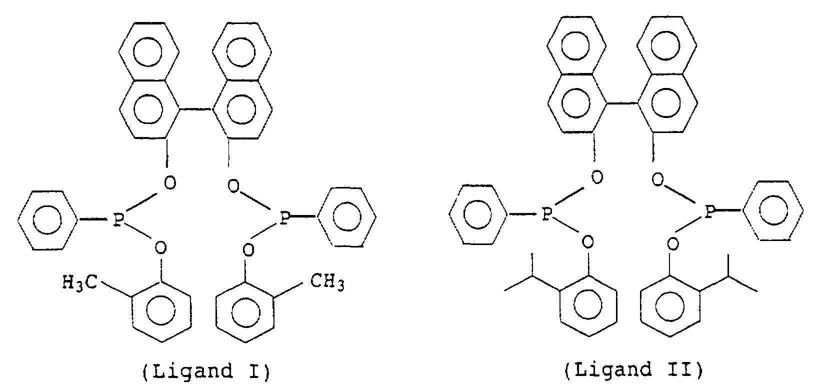

### Beispiel 1 (erfindungsgemäß):

### Semikontinuierliche Hydrocyanierung von 1,3-Butadien

In einem Glasautoklaven werden unter Argon bei Raumtemperatur 0,41 g (1,5 mmol) Bis(1,5-cyclooctadien)nickel(0), 2,14 g Ligand I und 10 ml Toluol vorgelegt und 10 Minuten gerührt, wobei sich der Reaktionsansatz rot-braun färbt. Danach wird eine Mischung aus 7,9 g (146 mmol) 1,3-Butadien und 40 g Toluol zugegeben. Der Glasautoklav wird fest verschlossen und die Reaktionsmischung auf 70 °C erhitzt, wobei sich ein Anfangsdruck von 1,2 bar einstellt. Über einen Zeitraum von 90 Minuten wird eine Mischung aus 3,2 g (118 mmol) frisch destillierter Blausäure in 40 g Toluol kontinuierlich zudosiert. Danach ist der Druck auf 0,5 bar gefallen. Anschließend lässt man zum Vervollständigen der Reaktion noch 120 Minuten bei etwa 70 °C nachreagieren. Zum Nachspülen des Reaktionsaustrages wird Toluol verwendet. Der Verlauf der Reaktion wird über Druck- und Temperaturmessung verfolgt.

Bei einer anschließenden Cyanid-Bestimmung nach Volhard wird ein Cyanwasserstoffumsatz von mehr als 99 % ermittelt.

GC-Analytik (Säule: 30 m Stabil-Wachs, Temperaturprogramm: 5 Minuten isotherm bei 50 °C, danach Aufheizen mit einer Geschwindigkeit von 5 °C/min auf 240 °C, Gaschromatograph: Hewlett Packard HP 5890) mit internem Standard (Benzonitril): 99,4 % 3-Pentennitril, 4-Pentennitril und 2-Methyl-3-butennitril, bezogen auf eingesetzten Cyanwasserstoff.

### Verhältnis 3-Pentennitril : 2-Methyl-3-butennitril = 0,41:1

Wie das folgende Beispiel 2 belegt, wird das Verhältnis von 3-Pentennitril zu 2-Methyl-3-butennitril durch eine Verlängerung der Reaktionszeit über das Ende der Cyanwasserstoffzugabe hinaus zugunsten von 3-Pentennitril verschoben. Der Zusatz eines Promotors ist nicht erforderlich.

### Beispiel 2 (erfindungsgemäß):

### Semikontinuierliche Hydrocyanierung von 1,3-Butadien mit Isomerisierung

In einem Glasautoklaven werden unter Argonatmosphäre bei Raumtemperatur 0,41 g (1,5 mmol) Bis(1,5-cyclooctadien)nickel(0), 2,9 g Ligand II und 10 g Toluol vorgelegt und 10 Minuten gerührt, wobei sich der Reaktionsansatz rot-braun färbt. Danach wird eine Mischung aus 8,1 g (150 mmol) 1,3-Butadien und 40 g Toluol zugegeben. Der Glasautoklav wird fest verschlossen und die Reaktionsmischung auf 90 °C temperiert. Über einen Zeitraum von 90 Minuten wird eine Mischung aus 4,0 g frisch destillierter Blausäure in 40 g Toluol kontinuierlich zudosiert. Nach Zulaufende wird die Temperatur auf 110 °C erhöht. Der Verlauf der Isomerisierung (Verhältnis von 3-Pentennitril zu 2-Methyl-3-butennitril) wird in regelmäßigen Abständen (0, 3, 6, 22 h) mittels GC-Analytik, wie in Beispiel 1 beschrieben, untersucht. Die Ergebnisse sind in Tabelle 1 wiedergegeben.

**Tabelle 1:**

| Zeit nach Zulaufende [h] | Verhältnis 3-Pentennitril : 2-Methyl-3-butennitril |
|---|---|
| 0 | 0,27:1 |
| 3 | 1,94:1 |
| 6 | 4,75:1 |
| 22 | 8,25:1 |

Da aufgrund der Entnahme der Proben für die Gaschromatographie eine exakte Ausbeutebestimmung nicht möglich war, wurde der gleiche Ansatz ohne Probenentnahme erneut durchgeführt. Eine Nachreaktionszeit erfolgte nicht.
Ausbeute: 99,6 %
Verhältnis 3-Pentennitril : 2-Methyl-3-butennitril = 0,22:1
(Ausbeutebestimmung: siehe Beispiel 1)

### Beispiel 3 (erfindungsgemäß):

### Isomerisierung von 2-Methyl-3-butennitril zu 3-Pentennitril

Unter Argonatmosphäre werden 0,72 g Ligand I, 15 ml Toluol und 0,14 g (0,5 mmol) Bis(1,5-cyclooctadien)nickel(0) vorgelegt und bei Raumtemperatur 45 Minuten gerührt. Dabei fällt aus der zunächst homogenen Lösung der sich bildende Katalysatorkomplex aus. Die flüchtigen Bestandteile werden im Hochvakuum entfernt. Der verbleibende Feststoff wird mit 40,5 g (500 mmol) 2-Methyl-3-butennitril versetzt. Die Lösung wird auf 110 °C erwärmt. Der Reaktionsverlauf wird in regelmäßigen Abständen mittels eines Gaschromatographen untersucht. Das Produktverhältnis nach 300 Minuten Reaktionszeit ist in Tabelle 2 angegeben. Alle dort verzeichneten Produkte und Nebenprodukte wurden zuvor mittels Gaschromatographie, GC-MS, GC-MS-IR sowie mittels NMR zugeordnet. Alle Werte sind in GC-Flächenprozent angegeben.
Einwaage Probe: 1,0160 g
Einwaage Standard: 1,4416 g

**Tabelle 2:**

| Produktverhältnis nach 300 Minuten Reaktionsdauer | |
|---|---|
| Verbindung | Anteil [GC-Flächen-%] |
| trans-2-Methyl-2-butennitril | 0,98 |
| 2-Methyl-3-butennitril | 7,41 |
| trans-2-Pentennitril | 0 |
| cis-2-Methyl-2-butennitril | 0,21 |
| 4-Pentennitril | 0,33 |
| trans-3-Pentennitril | 43,10 |
| cis-3-Pentennitril | 1,32 |
| Methylglutarnitril | 0,14 |
| Benzonitril (Standard) | 45,55 |

Umsatz: 71,65 %
Selektivität: > 99 % (Anmerkung: Bereits das Einsatzmaterial enthält ca. 1 % cis- und trans-2-Methyl-2-butennitril)

Wie Beispiel 3 belegt, ist mit den erfindungsgemäßen Katalysatoren eine Isomerisierung auch ohne Promotorzusatz möglich.

### Beispiel 4 (erfindungsgemäß):

### Isomerisierung von 2-Methyl-3-butennitril zu 3-Pentennitril

Unter Argonatmosphäre werden 0,39 g Ligand II, 8 ml Toluol und 0,07 g (0,25 mmol) Bis(1,5-cyclooctadien)nickel(0) vorgelegt und bei Raumtemperatur 30 Minuten gerührt. Dabei fällt aus der zunächst roten homogenen Lösung ein Teil des sich bildenden Katalysatorkomplexes aus. Die flüchtigen Bestandteile werden im Hochvakuum entfernt. Der verbleibende Feststoff wird mit 20,2 g (250 mmol) 2-Methyl-3-butennitril versetzt. Die Lösung wird auf 125 °C erwärmt. Der Reaktionsverlauf wird in regelmäßigen Abständen mittels eines Gaschromatographen untersucht. Das Produktverhältnis nach 300 Minuten Reaktionszeit ist in Tabelle 3 angegeben. Alle dort verzeichneten Produkte und Nebenprodukte wurden zuvor mittels Gaschromatographie, GC-MS, GC-MS-IR sowie mittels NMR zugeordnet. Alle werte sind in GC-Flächenprozent angegeben.
Einwaage Probe: 1,2109 g
Einwaage Standard: 1,00262 g

**Tabelle 3:**

| Produktverhältnis nach 300 Minuten Reaktionsdauer | |
|---|---|
| Verbindung | Anteil [GC-Flächen-%] |
| trans-2-Methyl-2-butennitril | 3,87 |
| 2-Methyl-3-butennitril | 2,16 |
| trans-2-Pentennitril | 0,36 |
| cis-2-Methyl-2-butennitril | 1,43 |
| 4-Pentennitril | 1,31 |
| trans-3-Pentennitril | 38,20 |
| cis-3-Pentennitril | 3,60 |
| Methylglutarnitril | 0 |
| Benzonitril (Standard) | 47,95 |

Umsatz 95,74 %

## Patentansprüche

1. Katalysator, umfassend einen Komplex eines Metalls der VIII. Nebengruppe, mit einem zweizähnigen Phosphonitliganden der allgemeinen Formel I 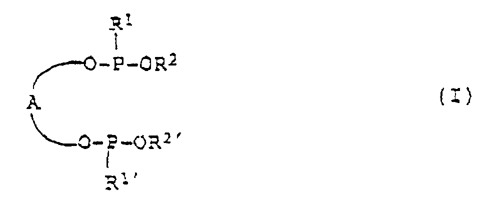 worin die Reste R¹ und R² bzw. R^{1'} und R^{2'} nicht miteinander verbrückt sind und
A für eine C₂- bis C₇-Alkylenbrücke steht, die eine, zwei oder drei Doppelbindungen und/oder einen, zwei oder drei Substituenten, die ausgewählt sind unter Alkyl, Cycloalkyl und Aryl, aufweisen kann, wobei der Arylsubstituent zusätzlich einen, zwei oder drei Substituenten, die ausgewählt sind unter Alkyl, Alkoxy, Halogen, Trifluormethyl, Nitro, Alkoxycarbonyl oder Cyano, tragen kann, und/oder die C₂- bis C₇-Alkylenbrücke durch ein, zwei oder drei nicht benachbarte, gegebenenfalls substituierte Heteroatome unterbrochen sein kann, und/oder die C₂- bis C₇-Alkylenbrücke ein-, zwei- oder dreifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E², tragen können, wobei E¹ und E² gleich oder verschieden sein können und für Alkyl, Cycloalkyl oder Aryl stehen,
R¹ und R^{1'} unabhängig voneinander für Alkyl, cycloalkyl, Aryl oder Hetaryl stehen, welche je einen, zwei oder drei Substituenten, die ausgewählt sind unter Alkyl, Cycloalkyl und Aryl, tragen können,
R² und R^{2'} unabhängig voneinander für Alkyl, Cycloalkyl, Aryl oder Hetaryl stehen, wobei die Aryl- und Hetarylaruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E^{2,} tragen können, wobei E¹ und E² die zuvor angegebenen Bedeutungen besitzen können,
oder Salze und Mischungen davon.

2. Katalysator nach Anspruch 1, wobei A für einen Rest der Formeln II.1 bis II.5 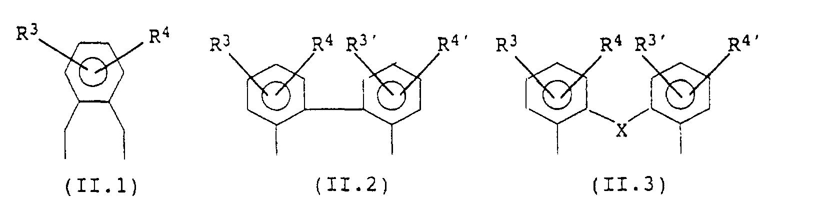 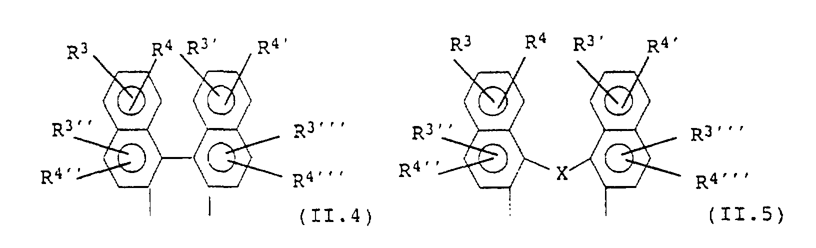 steht, worin
X für O, S, NR⁵ steht, wobei
R⁵ für Alkyl, Cycloalkyl oder Aryl steht,
oder X für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei Substituenten, die ausgewählt sind unter Alkyl, Alkoxy, Halogen, Trifluormethyl, Nitro, Alkoxycarbonyl oder Cyano, tragen kann,
oder X für eine C₂- bis C₃-Alkylenbrücke steht, die durch O, S oder NR⁵ unterbrochen ist,
R³, R^{3'}, R^{3''}, R^{3'''}, R⁴, R^{4'}, R^{4''} und R^{4'''} unabhängig voneinander für Wasserstoff, Alkyl, Alkoxy, Halogen, Trifluormethyl, Nitro, Alkoxycarbonyl oder Cyano stehen.

3. Katalysator nach einem der Ansprüche 1 oder 2, wobei R¹ und R^{1'} unabhängig voneinander für Alkyl oder Aryl, bevorzugt Phenyl, 1-Naphthyl oder 2-Naphthyl, stehen.

4. Katalysator nach einem der vorhergehenden Ansprüche, wobei R² und R^{2'} unabhängig voneinander für Phenylsubstituenten stehen, die gegebenenfalls einen oder zwei Substituenten, die ausgewählt sind unter Alkyl, Alkoxy, Halogen, Trifluormethyl, Nitro, Cyano, Alkoxycarbonyl oder Carboxyl, tragen können.

5. Katalysator nach einem der Ansprüche 1 bis 4, wobei der Phosphonitligand der Formel I ausgewählt ist unter Liganden der Formeln Ia bis Ic 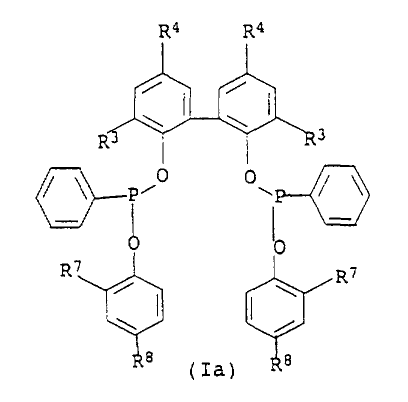 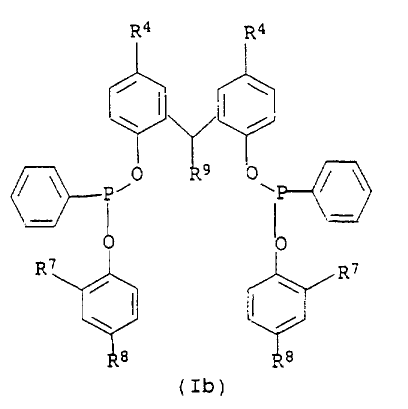 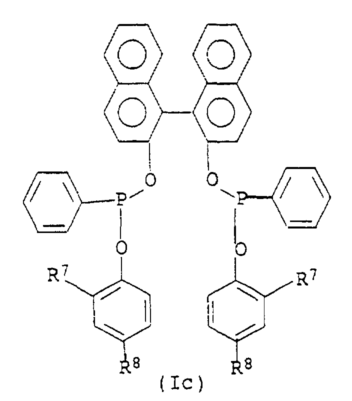 wobei in der Formel Ia die Substituenten R³, R⁴, R⁷ und R⁸ die folgenden Bedeutungen besitzen:
| R³ | R⁴ | R⁷ | R⁸ |
|---|---|---|---|
| H | H | H | H |
| tert.-Butyl | Methyl | H | H |
| tert.-Butyl | Methoxy | H | H |
| H | H | Methyl | H |
| H | H | Ethyl | H |
| H | H | Isopropyl | H |
| H | H | tert.-Butyl | H |
| H | Cl | H | H |
| H | CF₃ | H | H |
| H | H | Methyl | Methoxy |
in der Formel Ib die Substituenten R⁴, R⁷, R⁸ und R⁹ die folgenden Bedeutungen besitzen:
| R⁴ | R⁷ | R⁸ | R⁹ |
|---|---|---|---|
| H | H | H | H |
| Cl | H | H | H |
| Methoxy | H | H | H |
| H | H | H | Phenyl |
| H | Methyl | H | H |
| H | Methyl | Methoxy | H |
| H | Methyl | Methoxy | Phenyl |
in der Formel Ic die Substituenten R⁷ und R⁸ die folgenden Bedeutungen besitzen:
| R⁷ | R⁸ |
|---|---|
| H | H |
| Methyl | H |
| Ethyl | H |
| Isopropyl | H |
| tert.-Butyl | H |
| Methyl | Methoxy |
| i-Propyl | H |
| i-Propyl | Methoxy |
| H | Cl |
| H | CF₃ |

6. Katalysator nach einem der vorhergehenden Ansprüche, der zusätzlich wenigstens einen weiteren Liganden, ausgewählt unter Cyanid, Halogeniden, Aminen, Carboxylaten, Acetylaceton, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein-, zwei- und mehrzähnigen Phosphin-, Phosphinit- und Phosphitliganden aufweist.

7. Katalysator nach einem der vorhergehenden Ansprüche, wobei es sich bei dem Metall der VIII. Nebengruppe um Kobalt, Rhodium, Ruthenium, Palladium oder Nickel handelt.

8. Phosphonitliganden der allgemeinen Formel I 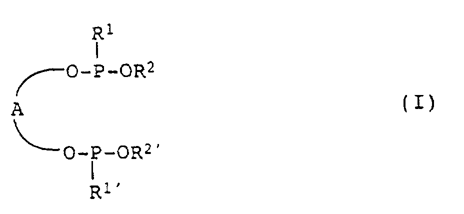 wie in einem der Ansprüche 1 bis 5 definiert, worin
R² und R^{2'} unabhängig voneinander für Alkyl, Cycloalkyl, Aryl oder Hetaryl stehen, wobei die Aryl- und Hetarylgruppen je einen oder zwei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E^{2,} tragen können, wobei E¹ und E² gleich oder verschieden sein können und für Alkyl, Cycloalkyl oder Aryl stehen.

9. Verfahren zur Herstellung von Gemischen monoolefinischer C₅-Mononitrile mit nichtkonjugierter C=C- und C=N-Bindung durch katalytische Hydrocyanierung von Butadien oder eines 1,3-Butadien-haltigen Kohlenwasserstoffgemisches, **dadurch gekennzeichnet, dass** die Hydrocyanierung in Gegenwart eines Katalysators nach einem der Ansprüche 1 bis 7 erfolgt.

10. Verfahren zur katalytischen Isomerisierung verzweigter aliphatischer Monoalkennitrile mit nichtkonjugierter C=C- und C ≡ N-Bindung zu linearen Monoalkennitrilen, **dadurch gekennzeichnet, dass** die Isomerisierung in Gegenwart eines Katalysators nach einem der Ansprüche 1 bis 7 erfolgt.

11. Verfahren zur Herstellung von Adipinsäuredinitril durch katalytische Hydrocyanierung von linearen monoolefinischen C₅-Mononitrilen, **dadurch gekennzeichnet, dass** die Hydrocyanierung in Gegenwart eines Katalysators nach einem der Ansprüche 1 bis 7 erfolgt.

12. Verfahren zur Herstellung von Adipinsäuredinitril, umfassend
a) Herstellung eines Gemisches monoolefinischer C₅-Mononitrile mit nichtkonjugierter C=C- und C≡N-Bindung durch katalytische Hydrocyanierung von Butadien oder eines 1,3-Butadien-haltigen Kohlenwasserstoffgemisches,
b) katalytische Isomerisierung des Gemisches aus a),
c) katalytische Hydrocyanierung des isomerisierten Gemisches aus b),
**dadurch gekennzeichnet, dass** die Schritte a), b) und c) in Gegenwart wenigstens eines Katalysators nach einem der Ansprüche 1 bis 7 und ohne Isolierung des/der Produkte(s) aus Schritt a) und/oder b) erfolgen.

13. Verwendung von Katalysatoren nach einem der Ansprüche 1 bis 7 zur Hydrocyanierung und/oder Stellungs- und Doppelbindungsisomerisierung von Olefinen.

## Claims

1. A catalyst comprising a complex of a metal of subgroup *VIII,* having a bidentate phosphonite ligand of the formula I 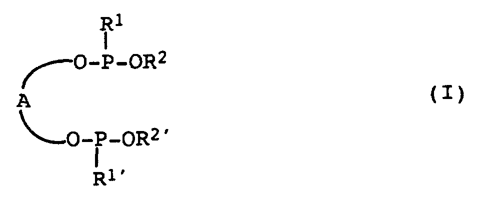 where R¹ and R², and R^{1'} and R^{2'}, are not linked to one another, and
A is a C₂- to C₇-alkylene bridge which may have 1, 2 or 3 double bonds and/or 1, 2 or 3 substituents which are selected from alkyl, cycloalkyl and aryl, it being possible for the aryl substituent additionally to carry 1, 2 or 3 substituents which are selected from alkyl, alkoxy, halogen, trifluoromethyl, nitro, alkoxycarbonyl and cyano, and/or the C₂- to C₇-alkylene bridge may be interrupted by 1, 2 or 3 non-neighboring, unsubstituted or substituted heteroatoms, and/or the C₂- to C₇-alkylene bridge may be fused with one, two or three aryl and/or hetaryl groups, it being possible for the fused aryl and hetaryl groups each to carry 1, 2 or 3 substituents which are selected from alkyl, cycloalkyl, aryl, alkoxy, cycloalkoxy, aryloxy, acyl, halogen, trifluoromethyl, nitro, cyano, carboxyl, alkoxycarbonyl and NE¹E², where E¹ and E² are identical or different and are each alkyl, cycloalkyl or aryl,
R¹ and R^{1'}, independently of one another, are each alkyl, cycloalkyl, aryl or hetaryl, each of which may carry 1, 2 or 3 substituents which are selected from alkyl, cycloalkyl and aryl,
R² and R^{2'}, independently of one another, are each alkyl, cycloalkyl, aryl or hetaryl, it being possible for the aryl and hetaryl groups each to carry 1, 2 or 3 substituents which are selected from alkyl, cycloalkyl, aryl, alkoxy, cycloalkoxy, aryloxy, acyl, halogen, trifluoromethyl, nitro, cyano, carboxyl, alkoxycarbonyl and NE¹E², where E¹ and E² may have the abovementioned meanings,
or a salt or mixture thereof.

2. A catalyst as claimed in Claim 1, A being a radical of the formulae II.1 to II.5 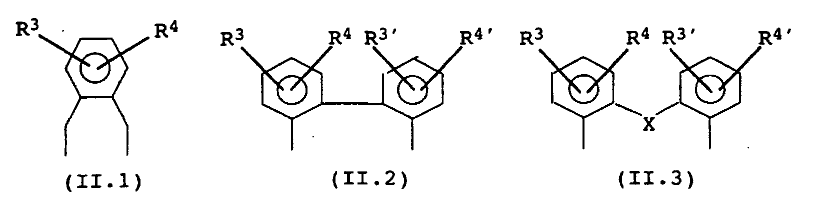 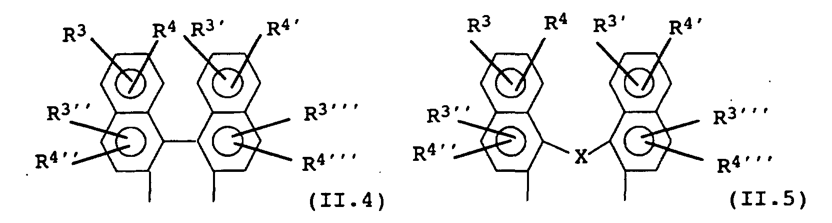 where
X is O, S or NR⁵, where
R⁵ is alkyl, cycloalkyl or aryl,
or X is a C₁- to C₃-alkylene bridge which may have a double bond and/or an alkyl, cycloalkyl or aryl substituent, it being possible for the aryl substituent to carry one, two or three substituents, which are selected from alkyl, alkoxy, halogen, trifluoromethyl, nitro, alkoxycarbonyl and cyano,
or X is a C₂- or C₃-alkylene bridge which is interrupted by O, S or NR⁵,
and
R³, R^{3'}, R^{3''}, R^{3'''}, R⁴, R^{4'}, R^{4''} and R^{4'''} independently of one another, are each hydrogen, alkyl, alkoxy, halogen, trifluoromethyl, nitro, alkoxycarbonyl or cyano.

3. A catalyst as claimed in either of claims 1 and 2, R¹ and R^{1'}, independently of one another, being alkyl or aryl, preferably phenyl, 1-naphthyl or 2-naphthyl.

4. A catalyst as claimed in any of the preceding claims, R² and R^{2'}, independently of one another, each being phenyl substituents which may carry one or two substituents which are selected from alkyl, alkoxy, halogen, trifluoromethyl, nitro, cyano, alkoxycarbonyl and carboxyl.

5. A catalyst as claimed in any of claims 1 to 4, the phosphonite ligand of the formula I being selected from ligands of the formulae Ia to Ic 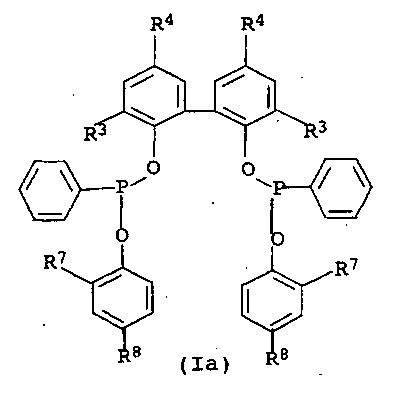 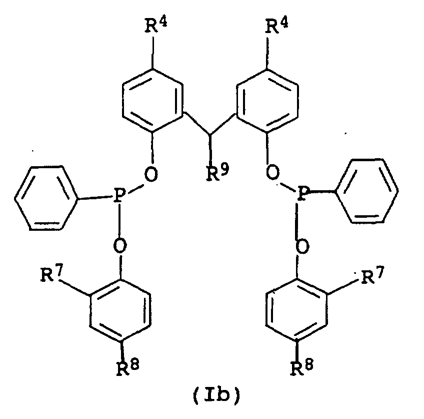 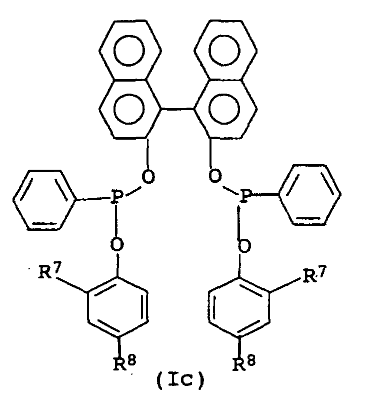 where, in the formula Ia, R³, R⁴, R⁷ and R⁸ have the following meanings:
| R³ | R⁴ | R⁷ | R⁸ |
|---|---|---|---|
| H | H | H | H |
| tert-butyl | methyl | H | H |
| tert-butyl | methoxy | H | H |
| H | H | methyl | H |
| H | H | ethyl | H |
| H | H | isopropyl | H |
| H | H | tert-butyl | H |
| H | Cl | H | H |
| H | CF₃ | H | H |
| H | H | methyl | methoxy |
in the formula Ib, R⁴, R⁷, R⁸ and R⁹ have the following meanings:
| R⁴ | R⁷ | R⁸ | R⁹ |
|---|---|---|---|
| H | H | H | H |
| Cl | H | H | H |
| methoxy | H | H | H |
| H | H | H | phenyl |
| H | methyl | H | H |
| H | methyl | methoxy | H |
| H | methyl | methoxy | phenyl |
in the formula Ic, R⁷ and R⁸ have the following meanings:
| R⁷ | R⁸ |
|---|---|
| H | H |
| methyl | H |
| ethyl | H |
| isopropyl | H |
| tert-butyl | H |
| methyl | methoxy |
| i-propyl | H |
| i-propyl | methoxy |
| H | Cl |
| H | CF₃ |

6. A catalyst as claimed in any of the preceding claims, which additionally has at least one further ligand selected from cyanide, halides, amines, carboxylates, acetylacetone, arylsulfonates, alkanesulfonates, hydride, CO, olefins, dienes, cycloolefins, nitriles, N-containing heterocycles, aromatics and heteroaromatics, ethers, PF₃ and mono-, bi- and polydentate phosphine, phosphinite and phosphite ligands.

7. A catalyst as claimed in any of the preceding claims, the metal of subgroup VIII being cobalt, rhodium, ruthenium, palladium or nickel.

8. A phosphonite ligand of the formula I 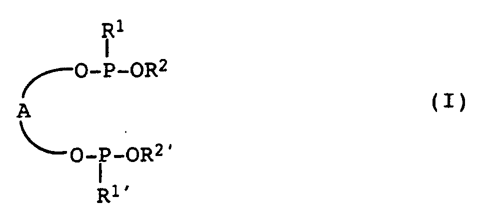 as defined in any of claims 1 to 5, where
R² and R^{2'}, independently of one another, are each alkyl, cycloalkyl, aryl or hetaryl, it being possible for the aryl and hetaryl groups each to carry one or two substituents which are selected from alkyl, cycloalkyl, aryl, alkoxy, cycloalkoxy, aryloxy, acyl, halogen, trifluoromethyl, nitro, cyano, carboxyl, alkoxycarbonyl and NE¹E², where E¹ and E² are identical or different and are each alkyl, cycloalkyl or aryl.

9. A process for the preparation of a mixture of monoolefinic C₅-mononitriles having a nonconjugated C=C and C ≡ N bond by catalytic hydrocyanation of butadiene or of a 1,3-butadiene-containing hydrocarbon mixture, wherein the hydrocyanation is carried out in the presence of a catalyst as claimed in any of claims 1 to 7.

10. A process for the catalytic isomerization of branched aliphatic monoalkenenitriles having a nonconjugated C=C and C≡N bond to give linear monoalkenenitriles, wherein the isomerization is carried out in the presence of a catalyst as claimed in any of claims 1 to 7.

11. A process for the preparation of adipodinitrile by catalytic hydrocyanation of a linear monoolefinic C₅-mononitrile, wherein the hydrocyanation is carried out in the presence of a catalyst as claimed in any of claims 1 to 7.

12. A process for the preparation of adipodinitrile, comprising
a) preparation of a mixture of monoolefinic C₅-mononitriles having a nonconjugated C=C and C ≡ N bond by catalytic hydrocyanation of butadiene or of a 1,3-butadiene-containing hydrocarbon mixture,
b) catalytic isomerization of the mixture from a), and
c) catalytic hydrocyanation of the isomerized mixture from b),
wherein the steps a), b) and c) are carried out in the presence of at least one catalyst as claimed in any of claims 1 to 7 and without isolation of the product or products from step a) and/or b).

13. The use of a catalyst as claimed in any of claims 1 to 7 for the hydrocyanation and/or positional and double-bond isomerization of olefins.

## Revendications

1. Catalyseur comprenant un complexe d'un métal du VIIIème sous-groupe, à base d'un ligand phosphonite bidenté de la formule générale I 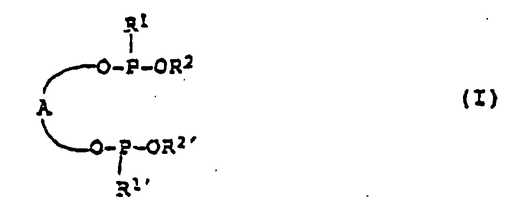 dans laquelle les restes R¹ et R², et R^{1'} et R^{2'} ne sont pas pontés ensemble et
A représente un pont d'alkylène en C₂ à C₇, qui peut présenter une, deux ou trois doubles liaisons et/ou un, deux ou trois substituants, choisis parmi des radicaux alkyle, cycloalkyle et aryle, le substituant aryle pouvant en outre porter un, deux ou trois substituants choisis parmi des radicaux alkyle, alcoxy, halogène, trifluorométhyle, nitro, alcoxycarbonyle ou cyano, et/ou les ponts d'alkylène en C₂ à C₇ peuvent être interrompus par un, deux ou trois hétéroatomes non voisins, éventuellement substitués, et/ou les ponts d'alkylène en C₂ à C₇ peuvent être annélés une, deux ou trois fois avec des radicaux aryle ou hétaryle, les groupes aryle ou hétaryle pouvant porter chacun un, deux ou trois substituants choisis parmi des radicaux alkyle, cycloalkyle, aryle, alcoxy, cycloalcoxy, aryloxy, acyle, halogène, trifluorométhyle, nitro, cyano, carboxyle, alcoxycarbonyle ou NE¹E², où E¹ et E² peuvent être identiques et différents et représentent des radicaux alkyle, cycloalkyle ou aryle,
R¹ et R^{1'} représentent indépendamment l'un de l'autre un radical alkyle, cycloalkyle, aryle ou hétaryle, qui peuvent porter un, deux ou trois substituants choisis parmi des radicaux alkyle, cycloalkyle et aryle,
R² et R^{2'} représentent indépendamment l'un de l'autre un radical alkyle, cycloalkyle, aryle ou hétaryle, où les groupes aryle et hétaryle peuvent porter chacun un, deux ou trois substituants choisis parmi des radicaux alkyle, cycloalkyle, aryle, alcoxy, cycloalcoxy, aryloxy, acyle, halogène, trifluorométhyle, nitro, cyano, carboxyle, alcoxycarbonyle ou NR¹E², où E¹ et E² peuvent avoir les significations indiquées ci-dessus,
ou bien leurs sels ou leurs mélanges.

2. Catalyseur selon la revendication 1, où A représente un reste des formules II.1 à II.5 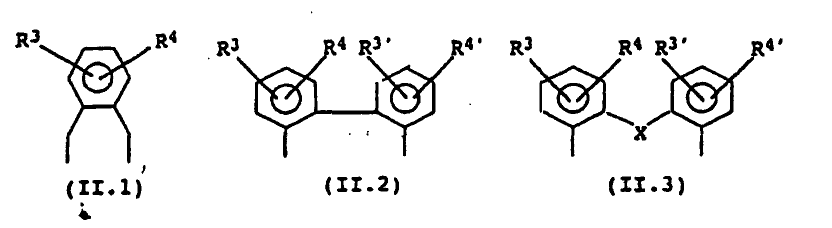 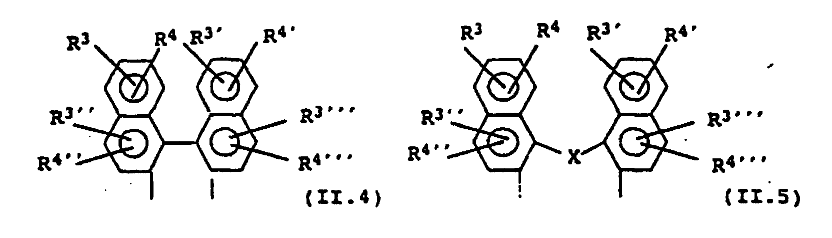 où
X représente O, S, NR⁵, où
R⁵ représente un radical alkyle, cycloalkyle ou aryle,
ou bien X représente un pont d'alkylène en C₁ à C₃, qui peut présenter une double liaison et/ou des substituants alkyle, cycloalkyle ou aryle, le substituant aryle pouvant porter un, deux
ou trois substituants choisis parmi des radicaux alkyle, alcoxy, halogène, trifluorométhyle, nitro, alcoxycarbonyle ou cyano,
ou bien X représente un pont d'alkylène en C₂ à C₃, interrompu par O, S ou NR⁵,
R³, R^{3'}, R^{3''}, R^{3'''}, R⁴, R^{4'}, R^{4''} et R^{4'''} représentent indépendamment les uns des autres de l'hydrogène ou un radical alkyle, alcoxy, halogène, trifluorométhyle, nitro, alcoxycarbonyle ou cyano.

3. Catalyseur selon la revendication 1 ou 2, où R¹ et R^{1'} représentent indépendamment l'un de l'autre un radical alkyle ou aryle, de préférence phényle, 1-naphtyle ou 2-naphtyle.

4. Catalyseur selon l'une des revendications qui précèdent, où R² et R^{2'} représentent indépendamment l'un de l'autre des substituants phényle, qui peuvent éventuellement porter un ou deux substituants choisis parmi des radicaux alkyle, alcoxy, halogène, trifluorométhyle, nitro, cyano, alcoxycarbonyle ou carboxyle.

5. Catalyseur selon l'une des revendications 1 à 4, où le ligand phosphonite de la formule I est choisi parmi des ligands des formules Ia à Ic 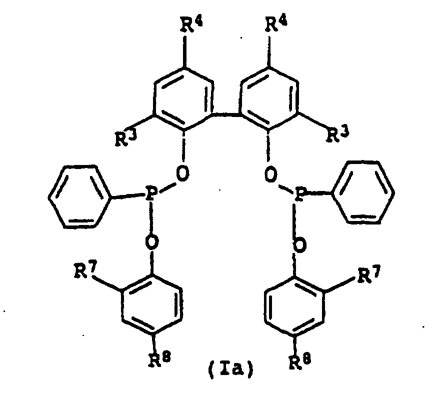 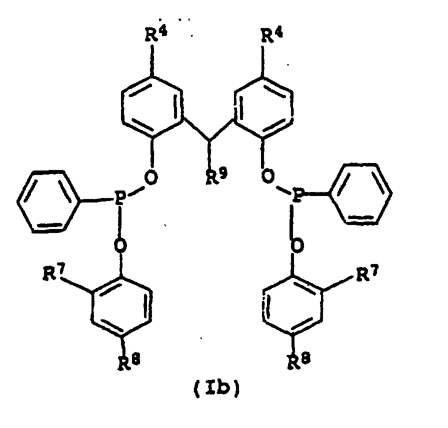 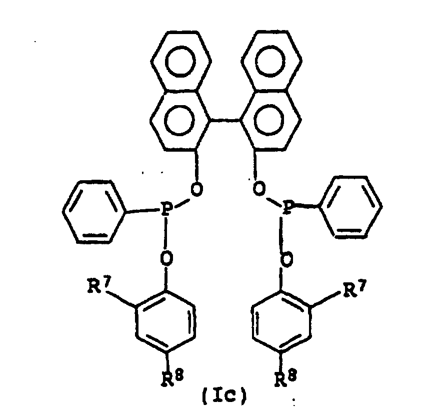 où, dans la formule la, les substituants R³, R⁴, R⁷ et R⁸ ont les significations suivantes:
| R³ | R⁴ | R⁷ | R⁸ |
|---|---|---|---|
| H | H | H | H |
| tert.-butyle | méthyle | H | H |
| tert.-butyle | méthoxy | H | H |
| H | H | méthyle | H |
| H | H | éthyle | H |
| H | H | isopropyle | H |
| H | H | tert.-butyle | H |
| H | Cl | H | H |
| H | CF₃ | H | H |
| H | H | méthyle | méthoxy |
dans la formule lb, les substituants R⁴, R⁷, R⁸ et R⁹ ont les significations suivantes:
| R⁴ | R⁷ | R⁸ | R⁹ |
|---|---|---|---|
| H | H | H | H |
| Cl | H | H | H |
| méthoxy | H | H | H |
| H | H | H | phényle |
| H | méthyle | H | H |
| H | méthyle | méthoxy | H |
| H | méthyle | méthoxy | phényle |
et dans la formule lc, les substituants R⁷ et R⁸ ont les significations suivantes:
| R⁷ | R⁸ |
|---|---|
| H | H |
| méthyle | H |
| éthyle | H |
| isopropyle | H |
| tert.-butyle | H |
| méthyle | méthoxy |
| i-propyle | H |
| i-propyle | méthoxy |
| H | Cl |
| H | CF₃ |

6. Catalyseur selon l'une des revendications qui précèdent, présentent en outre au moins un autre ligand, choisi parmi des cyanure, halogénure, amine, carboxylate, acétylacétone, aryl- ou alkylsulfonate, hydrure, CO, oléfine, diène, cyclooléfine, nitrile, hétérocycle azoté, aromatique et hétéroaromatique, éther, PF₃ ainsi que des ligands phosphine, phosphinite et phosphinate mono-, bi- et multidentés.

7. Catalyseur selon l'une des revendications qui précèdent, où le métal du Vlllème sous-groupe est du cobalt, du rhodium, du ruthénium, du palladium ou du nickel.

8. Ligands phosphonites de la formule générale I 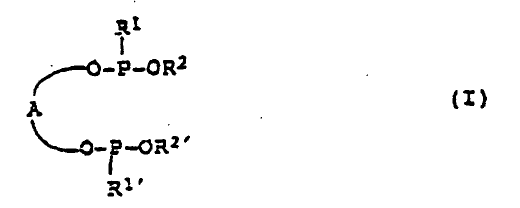 tels que définis dans l'une des revendications 1 à 5, où:
R² et R^{2'} représentent indépendamment l'un de l'autre un radical alkyle, cycloalkyle, aryle ou hétaryle, où les groupes aryle et hétaryle peuvent porter chacun un ou deux substituants choisis parmi des radicaux alkyle, cycloalkyle, aryle, alcoxy, cycloalcoxy, aryloxy, acyle, halogène, trifluorométhyle, nitro, cyano, carboxyle, alcoxycarbonyle ou NR¹E², où E¹ et E² peuvent être identiques ou différents et représenter des radicaux alkyle, cycloalkyle ou aryle.

9. Procédé de préparation de mélanges de mononitriles monooléfiniques en C₅ à liaisons C=C et C≡N non conjuguées, par hydrocyanuration catalytique de butadiène ou d'un mélange d'hydrocarbures contenant du 1,3-butadiène, **caractérisé en ce que** l'hydrocyanuration est opérée en présence d'un catalyseur selon l'une des revendications 1 à 7.

10. Procédé d'isomérisation catalytique de monoalcène-nitriles aliphatiques ramifiés à liaisons C=C et C≡N non conjuguées pour former des monoalcène-nitriles linéaires, **caractérisé en ce que** l'isomérisation est opérée en présence d'un catalyseur selon l'une des revendications 1 à 7.

11. Procédé de préparation de dinitrile adipique par hydrocyanuration catalytique de mononitriles monooléfiniques linéaires en C₅, **caractérisé en ce que** l'hydrocyanuration est opérée en présence d'un catalyseur selon l'une des revendications 1 à 7.

12. Procédé de préparation de dinitrile adipique, comprenant:
a) la préparation d'un mélange de mononitriles monooléfiniques en C₅ à liaisons C=C et C≡N non conjuguées, par hydrocyanuration catalytique de butadiène ou d'un mélange d'hydrocarbures contenant du 1,3-butadiène,
b) l'isomérisation catalytique du mélange de a),
c) l'hydrocyanuration catalytique du mélange isomérisé de b),
**caractérisé en ce que** les étapes a), b) et c) sont entreprises en présence d'au moins un catalyseur selon l'une des revendications 1 à 7 et sans isolation du(des) produits des étapes a) et b).

13. Utilisation des catalyseurs selon l'une des revendications 1 à 7 pour l'hydrocyanuration et/ou l'isomérisation de position et de double liaison d'oléfines.
